# FASCICULE DE BREVET EUROPEEN

(11) **EP 0 738 327 B1**
(45) Date de publication et mention de la délivrance du brevet: **21.10.1998**
(21) Numéro de dépôt: 95910583.4
(22) Date de dépôt: 22.02.1995
(51) Int. Cl.: C12N 15/86, C12N 15/87, A61K 48/00, C12N 9/12

(54) **SYSTEME HOTE-VECTEUR UTILISABLE EN THERAPIE GENIQUE**
WIRT-VEKTOR SYSTEM ZUR ANWENDUNG IN GENTHERAPIE
HOST-VECTOR SYSTEM FOR USE IN GENE THERAPY

(30) Priorité: 22.02.1994 FR 9401994; 15.12.1994 FR 9415135
(43) Date de publication de la demande: 23.10.1996
(60) Demande divisionnaire: 98103971.2
(73) Titulaire: UNIVERSITE PIERRE ET MARIE CURIE PARIS VI, 75252 Paris Cédex 05 (FR)
(72) Inventeur: KLATZMANN, David, F-75013 Paris (FR); SALZMANN, Jean-Loup, F-75011 Paris (FR)
(74) Mandataire: Desaix, Anne
(86) Numéro de dépôt international: FR9500208
(87) Numéro de publication internationale: WO9522617

(56) Documents cités:
- WO-A-88/01291
- WO-A-90/02176
- WO-A-90/02806
- WO-A-90/12087
- WO-A-91/19803
- WO-A-92/05266
- WO-A-93/04167
- US-A- 4 650 764
- VIROLOGY, vol.167, 1988 pages 400 - 406 MARKOWITZ ET AL. 'Construction and use of a safe and efficient amphotropic packaging cell line'
- MOL. CELL. BIOL., vol.7, no.5, Mai 1987 pages 1797 - 1806 BOSSELMAN ET AL. 'Replication-defective chimeric helper proviruses and factors affecting generation of competent virus : Expression of Moloney Leukemia Virus structural genes via the metallothionein promoter'
- PROC. NATL ACAD. SCI., vol.90, Août 1993 pages 7024 - 7028 CARUSO ET AL. 'Regression of established macroscopic liver metastases after in-situ transduction of a suicide gene'
- MOL. CELL. BIOL., vol.3, no.12, Décembre 1983 pages 2180 - 2190 JOYNER ET AL. 'Retrovirus transduction : generation of infectious retrovirus expressing dominant and selectable genes is associated with in-vivo recombination and deletion events'
- J. VIROLOGY, vol.66, no.6, Juin 1992 pages 3725 - 3732 TAKAHARA ET AL. 'A new retrovirus packaging cell for gene transfer constructed from amplified long terminal repeat-free chimeric proviral genes'
- HUMAN GENE THERAPY, vol.4, 1993 pages 461 - 476 RICH ET AL. 'Development and analysis of recombinant adenoviruses for gene therapy of cystic fibrosis'
- GENE, vol.119, no.2, 1 Octobre 1992 pages 265 - 272 NAHREINI ET AL. 'Cloning and integration of DNA fragments in human cells via the inverted terminal repeats of the adeno-associated virus 2 genome'

## Description

La présente invention est relative à une nouvelle approche pour la thérapie génique appliquée notamment à l'élimination de certaines catégories de cellules, telles les cellules tumorales ou certaines cellules infectées par des virus, par la production de rétrovirus recombinants à partir de nouvelles constructions génétiques.

Le concept de thérapie génique par gènes tueurs ou gènes suicides a été développé depuis 1986 par de nombreuses approches. Il implique l'expression d'un gène, cette expression permettant la transformation d'une substance non toxique ou substance toxique par la cellule. Ce concept de thérapie génique est applicable et transposable à tout gène dont l'expression dans une cellule cible permet de transformer une substance inactive en substance active, ou vice versa, conduisant soit à une destruction (exemple du gène suicide), soit à une restauration de certaines fonctions.

Dans tout ce qui suit, l'application de ces concepts aux gènes suicides est plus particulièrement développée compte tenu de l'accessibilité de sa mise en oeuvre expérimentale. La transposition des moyens de l'invention à tout type de transgène dont l'expression conduit à modifier l'effet d'une substance dans un sens d'activation ou d'inactivation, est néanmoins à la portée de l'homme du métier.

La thymidine kinase du virus Herpès Simplex de type I (HSV1-TK) est l'enzyme qui a été l'objet du plus grand nombre d'investigations relatives aux gènes suicides.

Cette enzyme, atoxique pour les cellules eucaryotes, présente la caractéristique de pouvoir transformer certains analogues nucléosidiques tels l'aciclovir (ACV) ou la ganciclovir (GCV) en molécules monophosphatées, ce dont sont normalement incapables les kinases cellulaires (G.B. Elion, J. Antimicrob. Chemother. 12:9-17 (1983)). Ces nucléosides monophosphates sont ensuite convertis par des enzymes cellulaires en nucléotides triphosphates qui peuvent être utilisés lors de la synthèse de l'ADN et bloquer le processus d'élongation entraînant ainsi la mort de la cellule. L'analogue de nucléoside triphosphate n'est donc toxique que pour les cellules en division.

On conçoit donc tous les avantages qui peuvent être tirés de l'utilisation de ce type de toxines conditionnelles appliquée à la thérapie génique, notamment anticancéreuse.

En premier lieu, seule une toxicité conditionnelle permet de générer des clones cellulaires stables produisant les pseudo-particules virales capables de produire un tel transgène et transférer le gène suicide dans les cellules cibles. Il suffit en effet de cultiver ces cellules en l'absence d'ACV ou de GCV puisque la thymidine kinase de l'HSV1 n'est pas toxique pour la cellule en absence de ces drogues.

Ensuite, en cas d'effet secondaire du traitement, l'arrêt de l'administration de l'ACV ou du GCV fait cesser immédiatement la toxicité due au transgène; en outre, un ajustement des doses de l'analogue nucléosidique permet de détruire sélectivement les cellules qui expriment fortement le transgène tout en préservant les cellules dans lesquelles le gène est faiblement exprimé ; cette toxicité restreinte aux cellules en division est un avantage important notamment pour le traitement des cellules cancéreuses.

Enfin, des données expérimentales in vitro et in vivo ont montré que des cellules n'exprimant pas HSV1-TK, mais au contact de celles-ci étaient également détruites par le traitement par l'ACV ("Coopération métabolique" ou "effet bystander")(Moolten F.L., 1986, Cancer Research, 46:5276-5281 et Culver K.W. et al., 1992, Science 256:1550-1552).

Le mécanisme de cet effet reste encore incompris, mais il est possible que les analogues nucléosidiques triphosphatés puissent passer d'une cellule à l'autre par l'intermédiaire des "gap junctions".

Les rétrovirus apparaissent comme étant des vecteurs de choix pour transférer des gènes exogènes dans des cellules eucaryotes, notamment des cellules humaines.

Cependant, un préalable indispensable pour l'utilisation des rétrovirus à des fins thérapeutiques et notamment en thérapie génique est de s'assurer de la sécurité de leur utilisation.

Le danger principal de l'utilisation de rétrovirus en thérapie génique est la possibilité d'une dissémination d'un rétrovirus sauvage reconstitué dans la population cellulaire ou le tissu considéré.

Une telle prolifération pourrait conduire à des intégrations multiples du génome du rétrovirus dans le génome des cellules infectées pouvant conduire à tout type de dérèglement génétique.

Ce type d'approche pose donc le problème d'atteindre le plus possible de cellules cibles, et donc la capacité de ce système d'atteindre le plus possible de cellules tumorales, tout en assurant le contrôle de la prolifération de virus.

De nombreuses approches ont été développées jusqu'à présent pour mettre au point des cellules d'empaquetage ("packaging cell lines") qui produisent uniquement des rétrovirus défectifs, et porteurs du transgène d'intérêt ; le développement de telles lignées cellulaires a augmenté de façon considérable l'utilisation des rétrovirus en thérapie génique, grâce à la sécurité conférée par ces systèmes.

Les premiers vecteurs utilisés ont été des vecteurs rétroviraux portés par des particules pseudo-rétrovirales ayant une enveloppe amphotrope pour permettre leur utilisation dans différentes espèces, et défectifs pour contrôler leur dissémination. Le virus naturel de départ est généralement le virus murin de Moloney dont on a séparé les éléments agissant en cis et les éléments agissant en trans pour former deux génomes défectifs.

Le vecteur viral proprement dit a conservé les séquences cis indispensables : les LTR pour le contrôle de la transcription et de l'intégration, la séquence psi nécessaire pour l'encapsidation, la séquence PB nécessaire pour la réplication virale. Les gènes viraux (gag, pol, env) sont délétés et remplacés par le transgène à exprimer dans une cellule cible placée en principe sous son propre promoteur ou sous un promoteur jugé plus puissant ou régulable.

Les gènes rétroviraux gag, pol, env sont souvent intégrés dans un autre vecteur, parfois appelé Helper, défectif pour les séquences LTR et Psi (ψ). Leur expression permet l'encapsidation du transgène, à l'exclusion des gènes nécessaires à la multiplication du génome viral et à la formation de particules virales complètes.

La forme provirale du Helper est en général intégrée dans le génome d'une lignée cellulaire murine (par exemple fibroblaste NIH/3T3) qui joue à la fois un rôle d'hôte pour le vecteur et de Helper pour les fonctions qui lui font défaut. Après transfection du vecteur, la souche cellulaire devient capable de produire des particules virales infectieuses défectives. Cependant, ces particules ne contiennent uniquement que le gène à transférer (transgène) mais ne contiennent pas l'information nécessaire à la reconstitution de particules virales complètes dans les cellules cibles. Ce système est donc conçu pour empêcher toute propagation ultérieure de virus après la première infection, c'est-à-dire si le virus infectieux porteur du transgène pénètre dans une cellule dépourvue de l'information de type Helper (gag, pol et env), sa production est arrêtée.

Donc, classiquement, la lignée d'empaquetage ("packaging cell line") est une lignée cellulaire susceptible d'apporter l'information Helper en trans par rapport au génome viral présent dans la particule, de telle façon que des particules virales infectieuses défectives puissent se reconstituer.

Différents systèmes d'empaquetage permettant la production de virus recombinants défectifs ont été décrits dans les documents suivants :
- la demande de brevet EP 0 243 204 décrit l'utilisation de rétrovirus comme moyen d'empaqueter toute substance pour traverser la membrane de cellules cibles eucaryotes ;
- la demande de brevet WO 89/07150 décrit une cellule de packaging de rétrovirus, les deux plasmides exprimant en trans les différents gènes viraux (env, gag, pol), mais aucun des deux ne comprenant la séquence Psi (ψ) de packaging ; la production de virus dans ces lignées est comprise entre 10⁴ et 10⁶ CFU/ml ;
- les demandes de brevets WO 90/02806, WO 90/12087, EP 0 476 953, WO 93/04167 et WO 93/10218 sont des publications décrivant des transferts de gènes in vivo par utilisation de vecteurs rétroviraux, eux-mêmes produits par des cellules d'empaquetage.

Les nombreuses références de constructions et d'applications citées dans ces brevets sont incluses à titre de références citées dans la présente demande.

Des développements récents ont conduit à utiliser un transfert de gènes in vivo en transférant directement les cellules d'empaquetage productrices de vecteurs pour le traitement de tumeurs ; l'élimination par exemple de tumeurs cérébrales expérimentales microscopiques par injection stéréotaxique de cellules produisant des rétrovirus TK-HSV1, puis traitement par le GCV, a été rapportée par Culver et col. (Science 256:1550-1552, 1992).

De la même façon, certains des auteurs de la présente demande ont montré que, chez le rat, le transfert in vivo du gène HSV1-TK par injection directe de fibroblastes murins produisant des particules rétrovirales recombinantes, conduit à une réduction considérable de tumeurs hépatiques chez le rat (Caruso M. et al., Proc. Natl. Acad. Sci. USA, 90:7024-7028, 1993 et Yves Panis et col., C.R. Acad. SC. Paris,, Tome 315, Série III, p. 541-544) ; ces articles mettent en outre en évidence une diminution très importante du nombre de cellules cancéreuses au niveau des tumeurs, sachant que, de façon surprenante, le nombre de cellules transduites in vivo par ce type de technique est sans doute inférieur à 10% pour TK-HSV1.

Cet effet pourrait s'expliquer par le mécanisme de coopération métabolique déjà évoqué plus haut.

Ce système de lignées d'empaquetage permettant de produire des particules virales porteuses d'un transgène est donc extrêmement prometteur pour utilisation en thérapie génique et notamment pour l'expression de gènes conditionnels.

Ces systèmes mis au point et décrits dans les nombreuses références citées plus haut présentent cependant certaines limites qui sont :
1) la faible productivité de ces lignées cellulaires (titre infectieux inférieur ou égal à 106 PFU/ml), le rétrovirus normal Mo MuLV produisant environ 108 PFU/ml et un virus comme l'adénovirus environ 109;
2) une deuxième limite de ce type de technique est qu'il met en oeuvre des lignées fibroblastiques NIH/3T3 qui sont des lignées adhérentes ; ceci est un avantage lorsque l'on utilise le surnageant de cellules contenant les particules virales, mais par contre est un inconvénient lorsque l'on veut utiliser les lignées transfectées elles-mêmes à titre de médicament, les lignées injectées in vivo produisant alors les particules virales recombinées in situ ;
3) un troisième inconvénient, qui est la contrepartie de l'avantage de sécurité du système, est que si les particules virales exprimées in situ par la lignée de packaging peuvent transférer le transgène d'intérêt dans les cellules en division, toute dissémination ultérieure est bloquée à ce niveau. Ceci limite l'efficacité du transfert du transgène du fait du blocage de la dissémination.

Il était donc important de concevoir un système permettant éventuellement d'auto-entretenir la production de particules virales recombinantes porteuses du gène d'intérêt tout en étant assurés de contrôler toute propagation ultérieure au moment choisi afin de préserver les conditions de sécurité requises, à savoir la maîtrise totale de la dissémination du virus sous sa forme sauvage.

Dans tout ce qui suit, le terme de "transgène" désigne le gène à exprimer dans la cellule cible, gène qui, dans les exemples est de type gène suicide.

Le trangène peut également être une cytokine ou une molécule d'intérêt thérapeutique.

L'expression "séquence pseudo-rétrovirale recombinante" signifie que la séquence comporte l'ensemble des gènes nécessaires à l'expression d'une particule pseudo-rétrovirale à l'exception du gène enveloppe (env) et comprenant éventuellement un ou plusieurs transgènes.

L'expression "cellule hôte" signifie la cellule contenant les deux séquences d'acides nucléiques recombinants, portées ou non par le même plasmide, et utilisable soit comme médicament, soit pour produire les virus recombinants défectifs.

L'expression "cellule cible" signifie la cellule que l'on cherche à traiter par introduction du transgène.

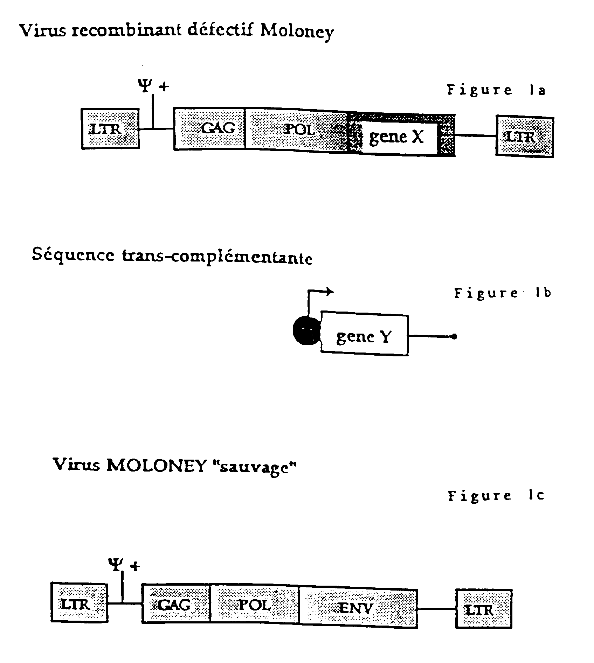

La présente invention répond et permet de surmonter les limites des systèmes existants citées ci-dessus par la construction d'un système hôte-vecteur permettant d'exprimer un transgène dans une cellule cible ou un tissu humain ou animal, caractérisé en ce qu'il est constitué d'une cellule eucaryote établie en lignée, dans laquelle ont été transfectées :
a) une séquence pseudo-rétrovirale recombinante dans laquelle le gène env a été délété dans sa totalité ou partiellement et substitué par ledit transgène au niveau du gène env tel que représenté sur la figure la, de sorte que le transgène soit exprimé sur des transcrits épissés similaires à ceux du gène env dans le virus Moloney sauvage;
b) une séquence d'acides nucléiques incluant une séquence codant pour une protéine d'enveloppe ou membranaire, laquelle séquence est sous la dépendance d'un promoteur et est associée le cas échéant audit transgène, et flanquée à son extrémité 3' un site de polyadénylation et représenté sur la figure 1b ; ledit génome viral recombinant et ladite séquence étant susceptibles de se complémenter en trans et permettre à ladite cellule hôte de produire des virus infectieux défectifs dépourvus du gène env.

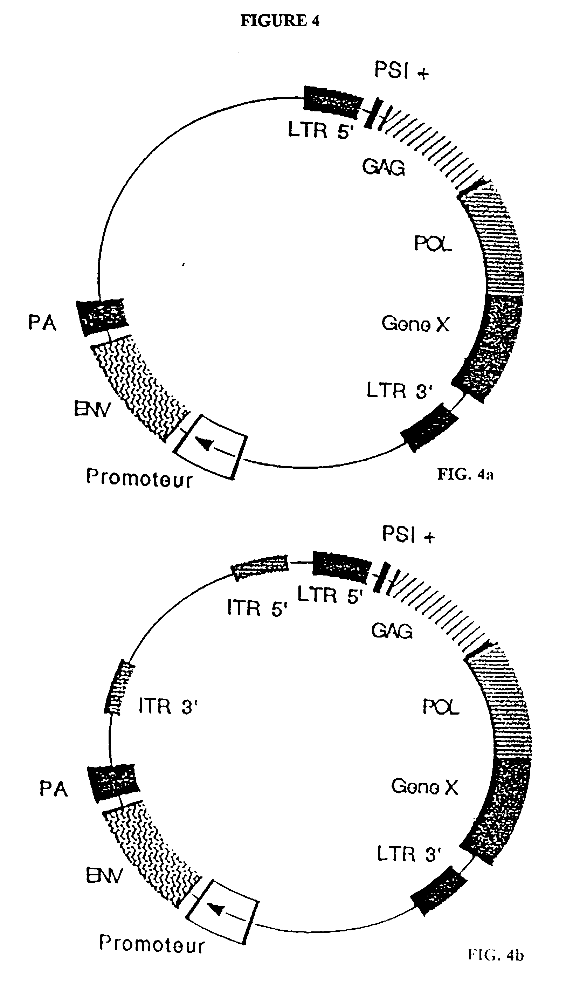

Le génome viral recombinant décrit en a) et la séquence d'acides nucléiques portant le gène env. décrite en b) peuvent être portés soit par deux plasmides ou systèmes de transfection différents, soit par le même plasmide ou système de transfection pourvu que la séquence b) soit située en dehors de la séquence pseudo-rétrovirale comprise entre les deux LTR. Une construction sur un seul plasmide est représentée sur la figure 4a.

La caractéristique commune aux deux types d'approches : deux supports distincts ou un seul support pour les séquences a) et b) est que la séquence b) étant dépourvue de la séquence ψ, elle n'est jamais empaquetée et par conséquent les virus produits par l'hôte en sont dépourvus.

En outre, dans ce dernier cas, le plasmide portant les deux séquences a) et b) ou celui portant la séquence pseudo-rétrovirale de la figure la ci-dessus peuvent porter d'autres séquences recombinantes destinées à améliorer la stabilité ou la durée de l'expression desdites séquences. Ces éléments complémentaires peuvent par exemple être les ITR (pour : Inverted Terminal Repeat) du virus AAV (Adeno Associated Virus). Un exemple d'une telle construction est représentée sur la figure 4b.

Il apparaît clairement dans ce système que la cellule hôte abritant les deux séquences recombinantes ci-dessus portées par un ou deux plasmides ne peut être assimilée à une cellule d'empaquetage dans la mesure où la première séquence pseudo-rétrovirale recombinante est porteuse de la séquence psi et permet, lorsqu'elle est transfectée seule, indépendamment de la deuxième séquence contenant le gène env, la production par la cellule hôte de particules virales porteuses de l'ensemble de la séquence de départ, mais qui sont dépourvues d'enveloppe et donc non infectieuses.

La cellule se comporte donc bien comme l'hôte d'un vecteur viral normal ou recombinant autorisant la production de particules virales sans adjonction de Helper ou de co-expression en trans comme décrit précédemment.

L'addition dans cette cellule hôte d'un gène codant pour la protéine d'enveloppe virale sous la dépendance d'un promoteur et suivie d'une séquence de polyadénylation permet la transcription, puis la traduction de ce gène en protéine dans la cellule hôte et, de façon subséquente, la reconstitution de particules virales porteuses du même génome que les précédentes (et représentée sur la figure la) à ceci près qu'elles possèdent désormais une enveloppe qui leur permet d'infecter des cellules cibles. Ces particules virales infectieuses ne sont, après infection d'une cellule cible, capables de produire des particules virales infectieuses que si le gène env est ajouté à nouveau et est exprimé.

Les plasmides recombinants de l'invention qui ont comme caractéristiques de posséder la séquence rétrovirale recombinante encadrée des deux LTR, et un gène env. porté ou non par le même support plasmidique, peuvent être également associés à un système permettant leur introduction dans les cellules cibles. Ces systèmes peuvent être de plusieurs types tels des virus ou un véhicule approprié favorisant le transfert du matériel génétique à l'intérieur des cellules ; les véhicules appropriés sont définis comme permettant le passage des membranes biologiques et peuvent être notamment des liposomes, des lipides cationiques, des dérivés de polylysine, des adénovirus inactivés ou des méthodes balistiques.

Des liposomes ont été utilisés pour envelopper et transférer dans des cellules aussi bien des acides nucléiques que des particules virales (R. Philips et al, (1994) Molecular and Cellular Biol. Vol 14 n° 4, p 2411-2418). Ces véhicules présentent une piètre efficacité de transfection des plasmides qu'ils contiennent. Le fait d'adjoindre à la séquence pseudo-rétrovirale de l'invention des séquences permettant une expression meilleure et prolongée des séquences d'intérêt thérapeutique permet d'envisager leur utilisation comme (trans)porteurs de plasmides recombinants soit comme moyen intermédiaire à la fabrication d'un médicament, soit comme principe actif de médicament ; la cellule ainsi transfectée par le véhicule contenant les vecteurs se transforme alors elle-même en cellule productrice de rétrovirus défectifs codant pour le gène d'intérêt mais dépourvus du gène enveloppe et le virus défectif est lui-même capable de réinfecter d'autres cellules pour y exprimer à nouveau le gène d'intérêt, le cycle s'arrêtant nécessairement à ce stade, sauf ajout d'une séquence env. par quelque moyen que ce soit. Cette cellule est soit la cellule du système hôte vecteur soit la cellule cible telle que définies plus haut.

L'invention est relative également aux véhicules tels que définis plus haut contenant une séquence pseudo-rétrovirale telle que décrite en la, une séquence recombinante portant un gène env. telle que décrite en 1b, séparés ou rassemblés sur un même support plasmidique tel que décrit dans les figures 4a et 4b, et portant en option des ITR du virus AAV en amont et en aval de la séquence recombinante pseudo-rétrovirale, et de préférence en aval de la séquence recombinante portant le gène env.

De manière plus générale, tout système de transfection dans lequel :
- un vecteur viral recombinant contenant un transgène d'intérêt, dans lequel un gène essentiel à la constitution de particules virales infectieuses est substitué par ledit transgène d'intérêt,
- ce gène essentiel est présent sur le même vecteur, ou un vecteur distinct, mais n'est plus sous la dépendance des promoteurs viraux et,
- le produit de ce gène agit en trans et permet de reconstituer des particules virales défectives, fait partie de l'invention.

Un autre exemple de construction de ce type, non rétrovirale, est la reconstitution d'un adénovirus recombinant défectif dans lequel le gène E1A est substitué par un transgène d'intérêt, et la séquence E1A est exprimée en trans de telle façon à reconstituer une particule virale infectieuse défective.

La caractéristique essentielle des constructions de l'invention, c'est qu'elles permettent de produire des virus infectieux soit dans une cellule hôte, soit dans une cellule cible, mais les virus produits contiendront un acide nucléique capable d'exprimer un transgène et incapable de produire des particules infectieuses.

Dans le cas de particules pseudo-virales décrites plus haut, la séquence env. sera en dehors de la zone de régulation de l'expression des séquences rétrovirales.

Ce système est particulièrement avantageux comme nous le verrons par la suite lorsque le transgène à exprimer est un gène suicide, notamment la thymidine kinase du virus Herpès Simplex (HSV1-TK), ou une cytokine.

Dans le système hôte-vecteur de l'invention, la séquence pseudo-rétrovirale recombinante est dérivée du génome du virus de Moloney MuLV, les séquences LTR en 5' ou 3' étant d'origine sauvage ou dérivées de différents mutants ou combinées. On pourra citer par exemple les constructions de type LTR dérivées des mutants mov3, mov9 et movl3 tels que décrits dans Caruso M. et al. dans Proc. Natl. Acad. Sci. USA, 90:7024-7028, 1993.

Cette séquence d'acides nucléiques (figure la), qui va être encapsidée dans la particule virale produite par la cellule hôte, est extrêmement proche du génome rétroviral "sauvage", ce qui présente l'avantage d'obtenir des titres plus élevés que ceux obtenus dans les systèmes décrits ci-dessus dans l'état de la technique avec les lignées de packaging et les complémentations en trans des gènes viraux.

A titre d'exemple, et comme nous le verrons plus loin, alors que dans les systèmes de l'état de la technique le titre maximum avec les cellules de packaging est de l'ordre de 106, ce système permet des titre compris entre 106 et 109 et de préférence entre 107 et 108 PFU/ml.

Une deuxième caractéristique du système hôte-vecteur de l'invention est que la cellule hôte est transfectée simultanément avec une séquence d'acides nucléiques incluant une séquence codant pour une protéine d'enveloppe, cette protéine étant d'origine virale, voire rétrovirale, ou protéine membranaire, voire cellulaire.

"Simultanément" signifie que les deux séquences, la séquence pseudo-rétrovirale et la séquence d'acides nucléiques codant pour le gène enveloppe, sont exprimées simultanément dans la cellule hôte, les transfections pouvant être réalisées soit simultanément sur une ou deux constructions, soit successivement.

Le gène enveloppe choisi peut être homologue à la séquence pseudo-rétrovirale, c'est-à-dire être issu par exemple du Mo-MuLV, permettant la reconstitution des particules pseudo-virales homologues ou être issu d'un autre virus, par exemple et sans être limitatif le virus de la stomatite vésiculaire (VSV), le HIV, le virus de la rage ou le virus de la leucémie du gibbon, lequel gène lorsqu'il est transcrit et traduit en protéine d'enveloppe permet la reconstitution d'un pseudo-type viral dont l'enveloppe est une enveloppe de VSV (ou de HIV) et le génome comportant les LTR, psi, PB, gag et pol du MuLV, ainsi que le transgène.

Enfin, le génome env peut être d'origine cellulaire et coder pour une protéine membranaire permettant le ciblage de la particule virale sur un ligand spécifique, notamment pour un récepteur de type CD4.

En outre, l'enveloppe peut être une protéine chimérique dont l'extrémité carboxyterminale est dérivée des séquences intramembranaires de l'enveloppe du Moloney. Il existe en effet des données expérimentales qui montrent que ces séquences membranaires peuvent être impliquées dans la concentration de ces protéines d'enveloppe à la surface de la particule virale. Avec ces molécules chimériques, l'efficacité d'expression de la protéine env chimérique à la surface des virions sera accrue.

Un deuxième avantage de ce système hôte-vecteur permettant l'expression de ces pseudo-types viraux hétérologues est la possibilité éventuelle de purifier ces pseudo-particules virales par ultracentrifugation sans perdre de pouvoir infectieux. Ceci permet d'obtenir des suspensions virales dont le titre peut atteindre 109 PFU/ml.

La séquence d'acides nucléiques contenant le gène env tel que décrit dans la figure 1b ci-après, peut être transfectée par tout type de moyens, que ce soient des moyens physiques ou à l'aide de vecteurs viraux ou rétroviraux. Parmi les moyens physiques connus, on pourra citer la micro-injection, les liposomes ou encore des procédés dits de biolistique ou par bombardement tels que décrits dans Kriegler, M. Gene Transfer and Expression; A Laboratory Manual; MacMillan Publishers Ltd.; 1990.

La transfection peut également avoir lieu par intégration de cette séquence d'acides nucléiques dans un vecteur viral et notamment de l'adénovirus.

L'avantage de cette dernière méthode est la possibilité de mettre en place un système auto-entretenu in vivo tout en gardant l'entière maîtrise de la prolifération virale.

En effet, le système hôte-vecteur comprenant comme transgène un gène suicide et injecté par exemple au niveau d'une tumeur va exprimer une particule virale recombinante qui, elle-même, va réinfecter in situ des cellules en division.

Ces cellules pourront à leur tour réexprimer une particule virale, mais, cette dernière sera non infectieuse puisque le génome viral n'aura pas été complémenté en trans par un gène d'enveloppe.

Si, à cette étape, il est procédé à une injection de vecteur d'expression recombinant contenant le gène env, il pourra y avoir à nouveau trans-complémentation et le cycle reproduit une fois de plus ; la sécurité du système est, de cette façon, préservée puisqu'une double transfection est nécessaire, l'une par la pseudo-particule virale MuLV susceptible d'infecter uniquement les cellules en division et l'autre par l'adénovirus susceptible d'infecter l'ensemble des cellules. Donc, seules les cellules en division seront susceptibles d'être co-transfectées, d'exprimer les gènes viraux en trans et de produire des pseudo-particules virales susceptible d'infecter les cellules cibles.

Ce système permet donc de façon particulièrement élégante de transfecter dans une cellule cible, par exemple le gène suicide TK, permettant l'expression de ce dernier et conférant à la cellule qui le contient la sensibilité aux analogues de nucléosides ; la transfection simultanée d'un gène enveloppe permet la production par cette même cellule cible de particules infectieuses comprenant le même génome pseudo-viral recombinant défectif en gène enveloppe.

Ce cycle peut donc être réitéré le nombre de fois désiré tant que la séquence porteuse du gène env est apportée de façon exogène ; le système s'arrête après un cycle réplicatif du virus dans la cellule cible, production de particules infectieuses, réinfection et production de particules non infectieuses.

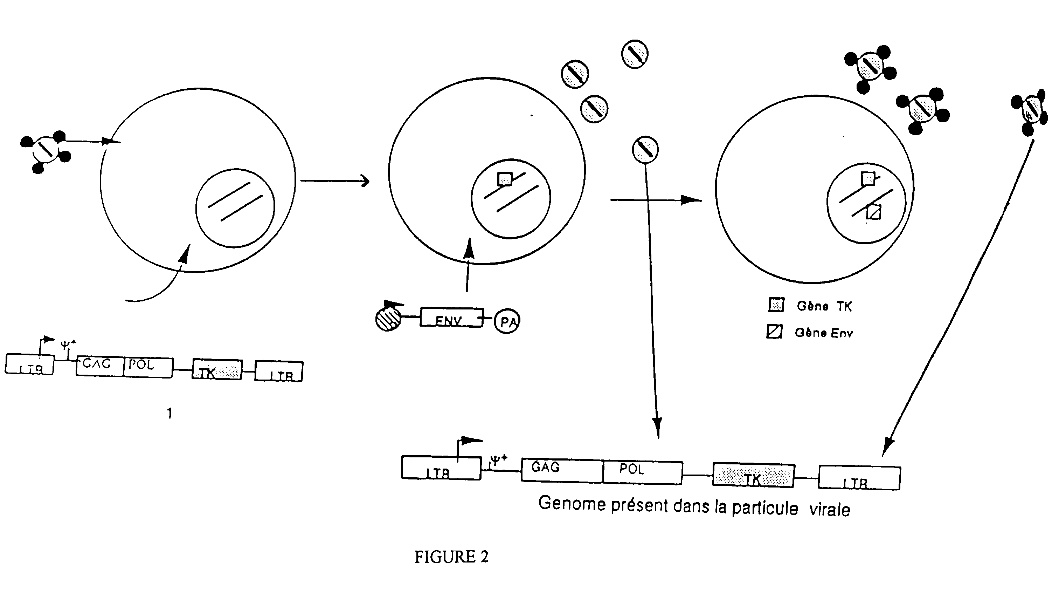

L'ensemble de ce cycle est décrit dans la figure 2.

Un troisième avantage du système hôte-vecteur selon l'invention est que tout type de cellules eucaryotes établies en lignées peut être choisi à partir du moment où cette lignée est susceptible de produire ce type de particules virales amphotropes.

Outre les lignées NIH/3T3 utilisées classiquement pour produire des particules virales recombinantes du virus de Moloney, le système hôte-vecteur de l'invention autorise l'utilisation de lignées cultivables en suspension ; à titre d'exemple, on peut citer les myélomes de souris, les cellules VERO ou les cellules d'insectes.

L'avantage évident d'utiliser des cellules en suspension est l'application industrielle pour la préparation de médicaments utilisables en thérapie génique, des quantités importantes de ces cellules étant nécessaires à de telles préparations qui sont bien évidemment beaucoup plus aisées d'obtention avec des cellules en suspension qu'avec des cellules adhérentes.

Le choix des cellules VERO comme cellules hôtes peut présenter l'avantage d'une part de provenir de primates et donc être mieux adaptées phylogénétiquement à l'homme et d'autre part d'être plus résistantes à l'action des anticorps naturels et du complément. Ces cellules ont enfin été utilisées largement pour la production de vaccins, et en particulier de virus qui ne subissent que très peu d'étapes d'inactivation ou de purification (vaccin contre la Rage par exemple). Certaines cellules utilisables à titre de cellules hôtes productrices de particules virales ont la propriété que lesdites particules ne sont pas inactivées par le complément : il s'agit notamment des lignées cellulaires humaines ou issues du vison.

Le choix comme cellules hôtes de cellules des lépidoptères ou plus généralement des cellules d'insectes (à l'exception des diptères) présente également de nombreux avantages pour construire des systèmes hôte-vecteur utilisables à titre de médicaments en thérapie génique:
- elles sont capables de produire des protéines d'enveloppe virales de virus infectant les eucaryotes, mais elles-mêmes ne sont pas infectables par ces virus ;
- les cellules d'insectes ne produisent pas de virus de mammifères, les tests de sécurité virale les concernant sont donc réduits ; ces cellules ne sont pas infectables par des virus potentiellement pathogènes pour l'homme ; il est donc beaucoup plus sûr d'utiliser ces cellules pour un usage en thérapeutique humaine sur le plan des tests de sécurité virale ;
- les cellules d'insectes poussent en suspension et donc sont cultivables à grande échelle ; les cellules d'insectes sont capables de déplacements intratissulaires ; aussi, dans le cas où elles seraient injectées au sein d'une tumeur, elles peuvent donc s'y mouvoir et délivrer leurs particules pseudo-virales à distance du lieu d'injection ;
- il est possible d'utiliser des promoteurs spécifiques aux cellules d'insectes qui sont des promoteurs forts : par exemple, il est possible d'utiliser des promoteurs de baculo virus qui donnent de forts taux d'expression et donc un nombreux accru de particules pseudo-virales ; étant donné qu'il y a plusieurs promoteurs forts issus de baculo virus, il est donc possible d'effectuer des constructions complexes afin de limiter les risques de recombinaison entre les différents gènes transfectés.

Tous ces avantages font que les cellules d'insectes, éventuellement modifiées génétiquement afin d'être capables de fabriquer des particules pseudo-virales amphotropes sont un outil de choix pour la construction du système hôte-vecteur de l'invention.

L'homme du métier saura, au cas par cas, selon le transgène qu'il souhaite exprimer dans une cellule cible, réaliser les constructions en utilisant les vecteurs d'expression appropriés et les cellules hôtes appropriées permettant la transcomplémentation du gène enveloppe avec le génome rétroviral porteur de la séquence psi, des gènes gag, pol et du ou des transgènes, et permettant une dissémination par l'adjonction du gène env, contrôlée dans le temps et dans l'espace.

Un autre perfectionnement avantageux du système hôte-vecteur de l'invention consiste à intégrer, dans la séquence d'acides nucléiques porteuse du gène env, une séquence du gène de la thymidine kinase de HSV1 ou tout autre gène suicide conditionnel ; ce perfectionnement est une deuxième clé permettant d'augmenter l'efficacité du traitement, ainsi que la sécurité du système, puisque, de cette manière, le gène suicide est exprimé dans la cellule cible sous la dépendance de promoteurs de types différents et, par conséquent, cela permet d'augmenter la sensibilité aux analogues de nucléosides conférée aux cellules par l'expression de ce gène suicide.

Un autre perfectionnement particulièrement avantageux pour des problèmes de sécurité virale dans une utilisation thérapeutique chez l'homme est l'utilisation d'un système hôte-vecteur dans lequel :
- les cellules hôtes sont des cellules d'insectes, non productrices de virus de mammifères ;
- le vecteur portant le gène env utilisé sous la dépendance d'un promoteur comporte en amont du gène env une séquence homologue du rétrovirus recombinant défectif utilisé dans le système hôte-vecteur, par exemple tout ou partie d'un gène gag ou tout ou partie d'un gène pol.

Ce système est intéressant car, si une recombinaison homologue avec un rétrovirus se produit dans un système où l'hôte comporte des rétrovirus endogènes, par exemple dans des cellules de souris, alors elle conduira à la production de rétrovirus non fonctionnels. Cette recombinaison aura lieu de telle sorte que le génome recombinant qui en résulte sera également défectif. L'injection de ce type de cellules à l'homme sera de toute sécurité sur le plan viral.

De même, l'utilisation avec ce système d'une cellule d'insecte dans le système hôte-vecteur ne peut conduire à des recombinaisons homologues, les cellules d'insectes ne portant pas de rétrovirus de mammifères et, par conséquent, l'injection à des fins thérapeutiques ne pourra permettre de produire que les particules virales recombinantes défectives de l'invention, à l'exclusion de particules virales issues d'une recombinaison homologue et qui pourrait alors être infectieuse.

La présente invention est également relative à un procédé d'expression d'un transgène pour la thérapie génique qui consiste en la mise en oeuvre des étapes suivantes :
a) construction d'un système hôte-vecteur par transfection dans une cellule eucaryote hôte, d'une part, d'une séquence pseudo-rétrovirale dans laquelle le gène env est délété dans sa totalité et remplacé par le transgène, par exemple au niveau de l'ATG dudit gène env et, d'autre part, une séquence d'acides nucléiques comportant dans sa structure une séquence codant pour une protéine d'enveloppe sous le contrôle d'un promoteur, le cas échéant associé au transgène et flanqué à son extrémité 3' d'une séquence de polyadénylation, les deux séquences ci-dessus étant portées par un ou deux vecteurs de type plasmidique,
b) mise en contact dudit système avec les cellules dans lesquelles le transgène doit être exprimé,
c) le cas échéant, transfert à nouveau dans les cellules cibles de la susdite séquence contenant le gène env. Dans le cas où les deux séquences sont portées par la même construction, la séquence comportant la séquence codant pour une protéine env. est extérieure aux deux LTR 5' et 3' de la séquence pseudo-rétrovirale.

Dans le procédé de l'invention, le transgène peut être un gène d'intérêt thérapeutique et notamment un gène suicide tel le gène de la thymidine kinase de HSV1 conférant, comme il a été dit plus haut, la sensibilité des cellules exprimant ce gène à des analogues de nucléosides.

Dans le procédé de l'invention, la séquence pseudo-rétrovirale est directement dérivée du virus de Moloney MuLV, les séquences LTR en 5' ou en 3' étant dérivées directement d'espèces virales particulières telles le mov9, mov3 ou mov13, elles peuvent être d'origine sauvage, mutante ou combinées.

Dans le procédé de l'invention, la séquence contenant le gène env est choisie notamment parmi les séquences de rétrovirus codant pour ledit gène et notamment le gène env du MuLV ou le gène env d'un virus hétérologue tel par exemple le VSV, le HIV, le virus de la rage, le virus de la leucémie du Gibbon. Il va de soi que la particule virale reconstituée est une particule hybride dont l'enveloppe est constituée de la protéine d'enveloppe du virus dont a été issue la séquence env et le génome viral étant issu du MuLV.

Le procédé selon l'invention est caractérisé par le fait que la séquence contenant le gène env (que cette séquence soit incluse dans un plasmide portant également la séquence pseudo-rétrovirale ou au contraire qu'elle soit incluse dans un vecteur autonome de cette dernière) est introduite dans la cellule cible par tout moyen approprié, notamment par un vecteur viral, tel l'adénovirus, ou encore par des méthodes physiques, par exemple bombardement, fusion de liposomes ou micro-injection. Il va de soi, comme cela a été expliqué plus haut, que l'utilisation d'un adénovirus permet la mise en oeuvre d'un procédé à haut rendement et tout en conservant une sécurité totale puisque l'expression de particules virales ne peut être réalisée que dans les cellules en division et par transcomplémentation de la pseudo-particule virale dérivée du virus de Moloney et du gène enveloppe dérivé du vecteur adénoviral.

Une variante avantageuse du procédé de l'invention, notamment quand les liposomes sont utilisés comme moyens de transfection, est l'addition d'une ou plusieurs séquences, à l'extérieur des deux LTR, dont la fonction serait une fonction stabilisatrice de l'expression de la séquence pseudo-rétrovirale et notamment du transgène.

Un exemple de ces séquences est les séquences ITR (Invented Terminal Repeat) du virus AAV. Ces séquences sont susceptibles, quand elles sont présentes de part et d'autre de séquences à exprimer, et notamment en amont, de conférer une stabilisation substantielle de l'expression desdites séquences (Philips et al, supra et Flotte T.T. et al. Am. J. Resp. Cell. Mol. Biol, 1992 7: 349).

Hors ce type de séquences, tout type de séquence susceptible d'augmenter l'expression en terme de taux et/ou de durée peut être choisie par l'homme du métier et intégrée dans le vecteur ou plasmide de transfection à l'extérieur des deux séquences nucléosidiques de type a) et b) ci-dessus.

De façon plus générale, l'invention est relative à un procédé permettant l'expression d'un transgène pour la thérapie génique caractérisé par la transfection simultanée dans des cellules cibles d'un génome rétroviral de structure générale représentée dans la figure la, lequel génome possède toutes les séquences nécessaires à l'expression d'une particule virale, mais est dépourvu du gène enveloppe conférant à ladite particule virale son caractère infectieux et, d'autre part, une séquence d'acides nucléiques contenant dans sa structure un gène enveloppe de virus ou cellulaire, la co-expression en trans des deux séquences ainsi transfectées permettant l'expression de particules virales recombinantes infectieuses mais dépourvues dans leur génome dudit gène env.

Cette co-expression en trans des deux types de séquences d'acides nucléiques peut être réalisée soit dans une cellule hôte permettant donc la réalisation d'un système hôte-vecteur utilisable en tant que tel en thérapie génique, soit en injection simultanée au niveau des cellules cibles à traiter, soit enfin par encapsulation dans un véhicule, par exemple des liposomes.

Cette expression peut être réalisée aussi bien quand les séquences sont portées par deux structures différentes, soit par la même structure plasmidique pourvu que celle portant le gène env. soit extérieure aux deux LTR 5' et 3'de la séquence pseudo-rétrovirale.

Il apparaît clairement à l'homme du métier le caractère itératif du système utilisé par adjonction en tant que de besoin de la deuxième séquence d'acides nucléiques porteuse du gène env et susceptible d'être exprimée sous le contrôle d'un promoteur.

L'invention est également relative à l'utilisation en thérapie génique du système hôte-vecteur décrit plus haut et obtenu par transfection simultanée dans une cellule eucaryote de deux séquences d'acides nucléiques dont l'expression en trans permet à ladite cellule eucaryote de produire des particules virales infectieuses et porteuses du transgène, cette utilisation pouvant être avantageusement appliquée à l'expression d'un gène suicide dans des cellules cibles à détruire.

Cette co-expression est réalisée par une double transfection de séquences décrites dans les figures la et 1b, ou par transfection d'une structure unique portant les deux types de séquences la et 1b, dont un exemple est représenté dans la figure 4a.

Si cette structure unique porte en outre une séquence améliorant l'expression en terme de taux ou de durée, telles les séquences ITR du virus AAV, l'utilisation est particulièrement avantageuse, notamment dans le cas de l'utilisation des liposomes comme moyen de transfection, améliorant ainsi l'efficacité de cette technique et permettant l'utilisation des liposomes porteurs des plasmides recombinants comme intermédiaire dans la fabrication d'un système hôte-vecteur utilisable en thérapie génique ou aussi comme principe actif de médicament utilisable en thérapie génique.

Les ITR d'AAV produisent leur effet maximal quand ils sont positionnés pour l'un en amont du LTR5' et pour l'autre en aval de la séquence de polyadénylation PA, telle que représentée dans la figure 4b.

Enfin, l'invention est relative à des médicaments utilisables en thérapie génique et caractérisés en ce qu'ils contiennent comme principe actif des cellules eucaryotes ayant subi une double transfection par les séquences d'acides nucléiques telle que décrite dans les figures 1a et 1b ; le principe actif d'un médicament selon la présente invention peut être également constitué, d'une part, de particules virales recombinantes infectieuses porteuses d'un génome viral dans lequel le gène env a été substitué par exemple au niveau de son codon d'initiation par le transgène à exprimer et, d'autre part, par une séquence d'acides nucléiques comportant dans sa structure le gène enveloppe, sous la dépendance d'un promoteur et, le cas échéant, flanquée par une séquence codant par exemple pour le gène de la thymidine kinase.

L'invention est de la même façon relative à des médicaments caractérisés en ce qu'ils contiennent comme principe actif des liposomes contenant des séquences d'ADN recombinantes dont l'une est constituée d'une séquence pseudo-rétrovirale comprise entre des LTR 5' et 3' de rétrovirus, comprenant les gènes gag et pol et un gène d'intérêt thérapeutique, mais dépourvu du gène env., et une autre séquence extérieure à la première comportant un gène env. et une séquence de polyadénylation et sous la dépendance d'un promoteur et le cas échéant également flanqué par une séquence codant pour un gène d'intérêt ; dans un mode de réalisation avantageux, le médicament constitué de ces liposomes porteurs des séquences recombinantes est tel que le plasmide qui les porte comprend en outre des séquences permettant d'améliorer l'expression des gènes d'intérêt et notamment ceux qui sont compris entre les deux LTR augmentant ainsi l'efficacité dudit médicament.

Le gène d'intérêt des médicaments de l'invention est de manière avantageuse le gène de la thymidine kinase, qui, quand il est exprimé, confère à la cellule qui l'abrite une sensibilité aux analogues de nucléotides tels que le gancyclovir ou l'acyclovir.

L'homme du métier saura mettre en oeuvre toutes les variantes possibles de ce type de système hôte-vecteur, par expérimentation de routine.

De telles variantes, ou équivalents, font partie de l'invention telle que revendiquée ci-après.

Les figures et les exemples qui suivent permettront d'illustrer de façon plus précise l'invention.
- La figure 1 représente la construction des deux séquences d'acides nucléiques devant être transfectées simultanément.
   Dans cette figure :
   . la figure 1a représente le virus recombinant défectif Moloney dans lequel la zone en gris représente des séquences dérivées du virus de Moloney et le gène X sur fond noir représente le transgène ;
   . la figure 1b représente un gène Y codant pour une enveloppe virale ou pour une protéine membranaire flanqué en 5' d'un promoteur et éventuellement d'une séquence de rétrovirus, et flanqué en 3' d'une séquence de polyadénylation ;
   . la figure 1c, pour mémoire, indique la séquence du virus Moloney sauvage.
- La figure 2 représente le principe de la co-expression en trans et de la production de particules virales défectives et infectieuses permettant la réintroduction dans une cellule cible du génome rétroviral recombinant défectif.

Dans la première partie du schéma, un vecteur de type représenté dans la figure la est introduit dans la cellule hôte. (Il est à noter que cette introduction peut être réalisée, soit par transfection, soit par infection par une particule rétrovirale défective telle que décrite à la fin de ce schéma). Le résultat de cette expression du vecteur représenté dans la figure la est la production de particules virales dont le génome est dérivé de ce vecteur mais qui ne portent pas à leur surface de protéine d'enveloppe et qui sont donc non infectieuses (telles que représentées dans la 2ème partie du schéma).

On exprime alors par transfection dans cette cellule un vecteur du type représenté dans la figure 1b, porteur du gène env. La complémentation en trans permet alors l'expression à la surface des particules virales de la protéine env, ces particules virales restant dépourvues du gène env.

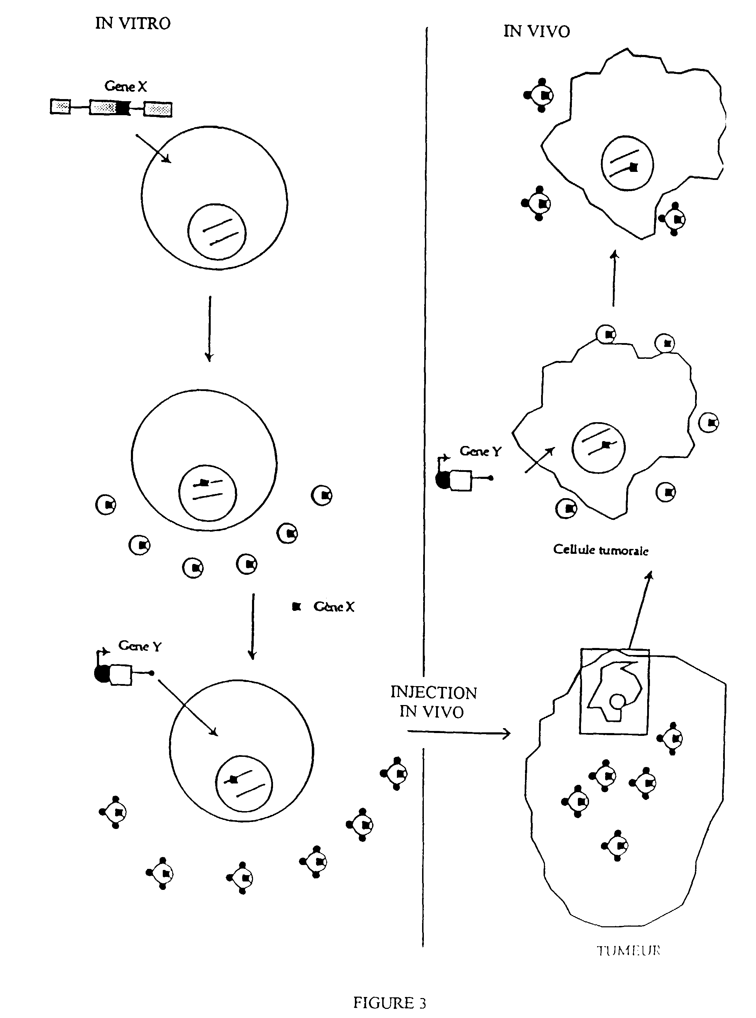

- La figure 3 représente le schéma thérapeutique depuis la génération des cellules produisant les particules virales infectieuses porteuses du transgène jusqu'à leur injection in situ dans la tumeur ainsi que la perpétuation du cycle infectieux par la complémentation in situ avec un gène d'enveloppe. La colonne de gauche reprend la production de particules virales recombinantes défectives telles que déjà représentées dans la figure 2, lesquelles cellules sont injectées in vivo dans une tumeur. Les particules virales recombinantes défectives produites in situ sont alors susceptibles d'infecter des cellules tumorales ; l'effet thérapeutique du transgène peut alors s'exprimer dans ces cellules et les cellules tumorales transduites produisent elles-mêmes des particules virales porteuses du transgène et dépourvues d'enveloppe. Le cycle s'arrête à ce niveau, sauf ajout ultérieur du vecteur selon la figure 1b.

La figure 4a représente une construction plamsmidique dans laquelle les deux séquences -pseudo-rétrovirales et env. - sont portées par la même structure.

La figure 4b est une variante dans laquelle les ITR du virus de l'AAV ont été ajoutés en amont du 5' LTR et en aval de la séquence de polyademylation (PA).

Le transgène est dans l'un et l'autre cas le gène de la thymidine kinase de HSV1.

La figure 5 représente un couple particulier de vecteurs utilisés pour montrer l'efficacité du système de l'invention, la figure 5a étant le vecteur pNP-2 contenant le gène HSV1-TK, et le plasmide p CRIP GAC-2 contenant le gène env du virus de Moloney, tels que décrits dans l'exemple 3 ci-après.

### - EXEMPLE I : Construction d'un virus de Moloney porteur d'un gène thérapeutique et défectif pour l'enveloppe virale :

Le vecteur utilisé est dérivé du vecteur rétroviral pMA 245, lequel est dérivé du virus de Moloney MuLV et a été construit à partir de fragments MuLV clonés à partir de différentes espèces de Mov (Mov-3, -9, et -13) telles qu'établies par Jaenisch et al., 1981, Cell 24:519-529.

La construction génétique est facilement réalisable par l'homme de métier. On part d'un plasmide porteur d'un provirus Moloney infectieux. Un tel plasmide, lorsqu'il est transfecté dans une lignée cellulaire adéquate (par exemple cellule NIH-3T3 de souris), donne naissance à des particules virales infectieuses porteuses du génome de Moloney sauvage dérivé de la construction génétique transfectée. Dans ce génome, on substitue le gène codant pour l'enveloppe par un gène codant pour la protéine d'intérêt thérapeutique. Cette construction est réalisée de sorte que ce gène est synthétisé à partir des mêmes transcrits épissés que le gène d'enveloppe "sauvage". Dans un souci d'efficacité de l'expression du gène d'intérêt thérapeutique, et également d'efficacité de la production de particules rétrovirales, on peut même substituer le gène d'intérêt thérapeutique de sorte que le codon ATG de celui-ci se trouve exactement à la position de l'ATG du gène env de Moloney. Le gène d'intérêt thérapeutique est plus volontiers dérivé de l'ADN complémentaire et ne comporte donc pas d'intron et est également dépourvu de séquence de polyadénylation. La construction génétique est telle qu'elle respecte donc l'épissage naturel du virus de Moloney, qu'elle respecte les cadre de lecture des gènes gag et pol et qu'elle respecte l'intégrité du LTR3' du virus de Moloney. L'ensemble de la construction génétique est réalisé selon les règles de l'art par digestion enzymatique de l'ADN puis ligation des fragments appropriés ; ces techniques sont décrites dans Maniatis et al., 1989, Molecular Cloning, A Laboratory Manual. Si nécessaire, elle utilise la PCR ou toute technique d'amplification appropriée pour générer des fragments génétiques aux extrémités desquels se trouvent les sites de restriction appropriés au clonage.

Si nécessaire également, les différents fragments génétiques composant le provirus infectieux Moloney peuvent être décomposés en fragments plus courts répartis sur les plasmides distincts, de sorte qu'ils soient manipulés plus simplement pour être ensuite réassemblés de façon appropriée. Si nécessaire enfin, on peut utiliser la mutagénèse dirigée pour introduire, soit des sites de restriction nécessaires à la réalisation de la construction génétique, soit pour substituer à des positions précises les différents gènes. A l'issue de cette construction génétique, on dispose d'un plasmide qui, lorsqu'il est transfecté dans une cellule (par exemple NIH-3T3), s'exprime comme un virus de Moloney sauvage et aboutit donc à la génération de particules virales dont le génome est constitué par le provirus lui-même. Du fait de l'homologie entre le provirus infectieux et le provirus défectif nouvellement construit, la synthèse des différents ARN se fait avec une efficacité dans des proportions optimisées qui font que le nombre de particules virales qui sont fabriquées par la cellule est très voisin de celui d'un virus sauvage. Ce nombre de particules virales est donc largement supérieur à celui fabriqué par des lignées d'encapsidation classique dans lesquelles les différents gènes de structure du virus de Moloney sont portés par des constructions génétiques différentes, et dans laquelle le vecteur rétroviral qui constitue le génome de la particule virale est sur un plasmide différent et a une structure et une taille très différentes de celles du Moloney sauvage. Il est à noter que les particules virales ainsi produites ne sont pas infectieuses car elles sont dépourvues d'enveloppe. Cette protéine d'enveloppe peut être apportée de façon indépendante dans cette cellule grâce à un vecteur dont la construction est effectuée ci-après.

### - EXEMPLE II : Construction de la séquence d'acides nucléiques porteuse du gène env :

On peut en effet transfecter dans cette cellule une nouvelle construction génétique simple contenant un gène à expression membranaire sous le contrôle d'un promoteur classique (comme par exemple le promoteur du virus SV40, un promoteur de cytomégalovirus, un promoteur d'un gène housekeeping comme le PGK, ou bien même un promoteur spécifique d'un type cellulaire donné et qui offrira donc une sécurité d'emploi supplémentaire). Ce vecteur d'expression comprend également des séquences de polyadénylation provenant de différentes constructions génétiques (séquences de polyadénylation de SV40, de βglobine, de l'hormone de croissance...). Le gène membranaire peut être soit une enveloppe du virus Moloney lui-même, soit une enveloppe d'autre virus qui puisse être captée par la particule virale et exprimée à la surface de celle-ci, soit même une protéine cellulaire membranaire qui puisse également être présente à la surface de la particule virale. C'est le cas notamment de la molécule CD4 dont il a été montré qu'elle pouvait être incorporée par différents rétrovirus.

Un autre mode de réalisation est la construction des protéines membranaires chimériques, telles que leur partie intracellulaire est dérivée de la partie intracellulaire de l'enveloppe du virus Moloney, et leur partie extracellulaire est une protéine membranaire permettant de mieux cibler la particule virale. La présence des régions intracellulaires de l'enveloppe du Moloney assure probablement une meilleure efficacité d'expression de cette enveloppe chimérique à la surface de la particule virale.

Cette deuxième construction génétique est également réalisée de sorte qu'elle ne contienne aucune séquence dérivée d'un virus de Moloney autre que le gène enveloppe lui-même. Plus précisément, il ne peut contenir de séquence qui n'ait pas au préalable été délétée sur la construction génétique précédente. De la sorte, on minimise fortement la possibilité d'une recombinaison entre ce vecteur d'expression et le rétrovirus défectif tel qu'il a été décrit précédemment, recombinaison qui pourrait aboutir à la production d'un rétrovirus de Moloney infectieux cette fois-ci. Ces constructions génétiques sont également réalisées selon les règles de l'art comme indiqué précédemment.

### - EXEMPLE III : Expression de particules virales par transcomplémentation des deux séquences construites respectivement dans les exemples I et II :

Les constructions sont utilisées pour infecter des cellules de myélomes p3X63Ag8 ou des cellules 3T3 TK⁻ déficientes en thymidine kinase.

L'expérience ci-dessous comprend deux expériences contrôle et deux essais :
lot a) : un premier contrôle de milieu de sélection, dans lequel les cellules ne sont pas transfectées,
lot b) : un deuxième contrôle de l'efficacité de la transfection dans lequel les cellules sont transfectées avec un plasmide contenant un gène codant pour la βgalactosidase (Lac Z)
lot c) : un essai dans lequel les cellules sont transfectées par le plasmide pNP-2 seul mais qui permet l'expression de TK mais pas la propagation via la fabrication de particules rétrovirales recombinantes défectives,
lot d) : un essai dans lequel les cellules sont co-transfectées par le vecteur pNP-2 et un plasmide porteur du gène env du virus de Moloney Mo-MuLV.

### III.1 Description des plasmides

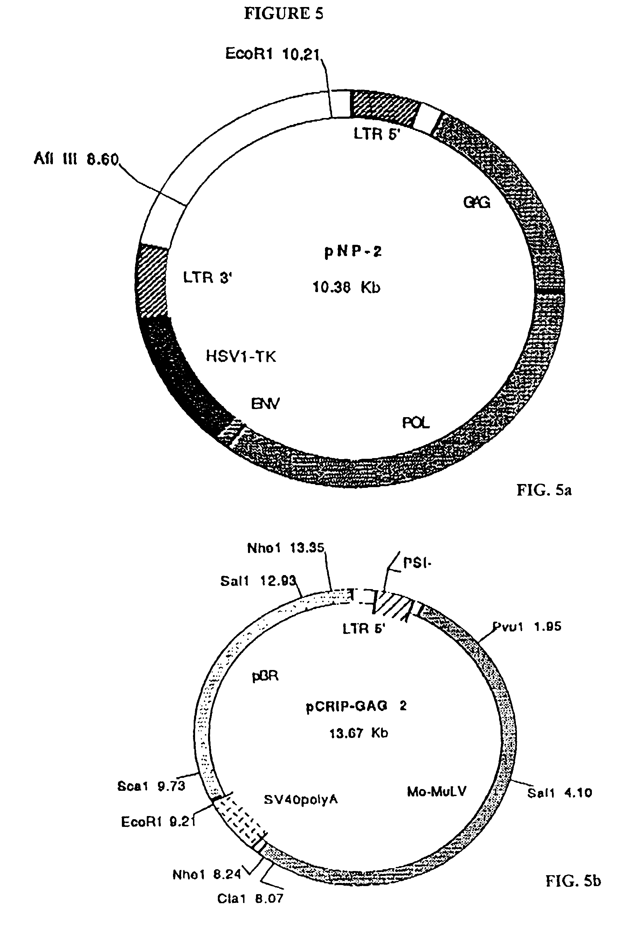

- La figure 5a représente le plamside pNP-2 qui a été déposé à la CNCM le 20 février 1995 sous le n°I-1541. Il contient les gènes GAG et POL de Mo-MuLV, avec en aval un gène HSV1-TK sous un ATG de gène env muté. Sa taille totale est 10.38 KB. Les sites AF III (à 8.60 Kb) et ECoR1 (à 10.21 KB) sont figurés.
- la Figure 5b représente le deuxième plasmide pCRIP GAG⁻², qui est un exemple de construction dans lequel le gène env du MoMuLV est intégré dans une séquence comportant les séquences MoMuLV, ψ- gag-, pol+, env+, et suivie en aval par une séquence polyA de SV 40. Les chiffres entre parenthèse indiquent la distance en nombre de paires de base.

### III.2 Transfection

Des cellules 3T3 TK sont soit non transfectées (lot a)), soit transfectées selon les conditions décrites plus haut (lots b), c) et d)).
Dans le lot b), l'efficacité de transfection est révélée par utilisation de substrat chromogène ; pour cela, les cellules sont cultivées en présence d'IPTG (induction de l'opéron Lac) et au bout de 24h en présence de x gal dont la couleur passe au bleu quand il est clivé par la βgalactosidase exprimée. La présence de la couleur bleue dans les cellules révéle donc l'existence de la transfection.
Dans les lots c) et d), les cellules sont mises en présence du milieu de sélection HAT soit à 24h, soit après 5 jours de culture où l'on s'attend à avoir un nombre de cellules résistantes sous HAT largement supérieur, compte tenu de la propagation cellulaire.

### III.3 Résultats

1) Efficacité de transfection : dans les conditions utilisées, l'efficacité de transfection avec le plasmide lac Z montre des valeurs aux alentours de 5% de cellules transfectées.
2) Clones obtenus après une sélection dès la 24ème heure. Pour les transfections avec le plasmide pNP-2 seul, on voit moins d'une dizaine de clones de toute petite taille (entre 20 à 50 cellules par clone). Pour la transfection avec pNP-2 plus le gène codant pour l'enveloppe de Moloney, on voit également une dizaine de clones de petite taille plus 38 clones de taille largement supérieure (>200 cellules par clone).
3) Clones obtenus après une sélection HAT au 5ème jour. Pour les transfections avec pNP-2 seul, on retrouve quelques clones également de petite taille. Pour la boîte correspondant à la transfection de pNP-2 et le plasmide env, on observe un nombre de clones très important, incomptable, correspondant environ au quart de confluence sur l'ensemble de la boîte.

### III.4 Conclusions

1) Le plasmide pNP-2 est fonctionnel et permet l'expression du gène TK.
2) Dès la 24ème heure, des particules virales recombinantes sont produites et permettent de transférer efficacement et de façon stable le gène TK dans les cellules de la culture.
3) Après 5 jours, on observe une augmentation importante (mais difficilement quantifiée dans cette expérience) du nombre de clones stables obtenus.

### - EXEMPLE IV : Construction d'un plasmide unique portant à la fois la séquence pseudo-rétrovirale recombinante dépourvue du gène env. et porteuse de la thymidine kinase de HSV1, et d'un gène env. sous la dépendance d'un promoteur en amont et d'un gène env.en aval.

Un plasmide classique est utilisé à titre de squelette de base, par exemple le PBR 322 par les méthodes décrites plus haut. Les constructions telles que représentées dans les figures 4a et 4b sont réalisées, le plasmide ainsi recombiné peut être ainsi émulsionné et enveloppé dans des liposomes ou associé à tout autre vecteur permettant de le faire pénétrer dans la cellule hôte ou dans la cellule cible.

### - EXEMPLE V : Résultats expérimentaux obtenus sur des tumeurs établies du système hôte-vecteur obtenu avec les constructions décrites dans les exemples I, II et III :

Il a été constaté que ce système hôte-vecteur ainsi construit produit des particules rétrovirales infectantes à un titre de 108 particules/ml.

Le titre a été vérifié par infection de cellules L- de souris déficientes en thymidine kinase et infectées selon la méthode décrite dans Caruso M. et al., Proc. Natl. Acad. Sci. USA, 90:7024-7028, 1993.

En conclusion, le procédé de traitement de cellules in vivo par thérapie génique à l'aide du système hôte-vecteur et les médicaments contenant à titre de principe actif ce système apparaît comme étant particulièrement avantageux au regard des techniques préexistantes sur les différents points cités plus haut, à savoir :
- la productivité des particules virales émises (au moins 1 à 2 logs au-dessus de la productivité des cellules de packaging classiques),
- le caractère opérationnel au niveau d'une mise en oeuvre industrielle de la production d'un médicament utilisant ce système puisque ce système hôte-vecteur peut utiliser des cellules hôtes cultivables en suspension et donc à grande échelle,
- une augmentation de l'efficacité du système au niveau des cellules cibles puisque la production de particules virales infectieuses porteuses du transgène est un processus itératif qui peut être répété in vivo par adjonction à l'aide d'un vecteur ou de tout moyen approprié d'une séquence d'acides nucléiques porteuse du gène env et sous la dépendance de son promoteur ; le système peut être bloqué permettant de stopper toute dissémination de particules virales recombinantes par arrêt de l'adjonction du gène env ; cela permet de contrôler, dans le cas de gènes suicides par exemple, le nombre de cellules détruites par adjonction d'analogues de nucléosides en fonction de la taille d'une tumeur éventuelle ou du nombre de cellules à détruire,
- enfin, ce système permet une grande souplesse d'utilisation tout en gardant une sécurité virale et peut être appliqué à tout autre système d'expression conditionnel de gène.

## Revendications

1. Système hôte-vecteur permettant d'exprimer un transgène dans une cellule cible ou un tissu humain ou animal caractérisé en ce qu'il est constitué d'une cellule eucaryote et établie en lignée, dans laquelle ont été transfectées :
a) une séquence pseudo-rétrovirale recombinante dans laquelle le gène env a été délété dans sa totalité ou partiellement et substitué par ledit transgène au niveau du gène env tel que représenté dans la figure 1a;
b) une séquence d'acides nucléiques incluant une séquence codant pour une protéine d'enveloppe, laquelle séquence est sous la dépendance d'un promoteur et est flanquée à son extrémité 3' d'un site de polyadénylation, telle que représentée dans la figure 1b ;
ledit génome viral recombinant et ladite séquence étant susceptibles de se complémenter en trans et permettre à ladite cellule hôte de produire des virus infectieux défectifs dépourvus du gène env.

2. Système selon la revendication 1 caractérisé en ce que la séquence en a) et la séquence en b) sont portées par deux systèmes de transfection distincts.

3. Système selon la revendication 1 caractérisé en ce que les séquences en a) et en b) sont portées par le même système de transfection pourvu que la séquence 1b) soit extérieure à la zone de régulation de l'expression de la séquence pseudo-rétrovirale recombinante en a), en particulier extérieure aux LTR.

4. Système selon la revendication 3 caractérisé, en ce que le système de transfection comporte en outre une ou deux séquences ITR de AAV situées en amont, ou en amont et en aval des LTR.

5. Système selon l'une des revendications 1 à 4, caractérisé en ce que la séquence d'acides nucléiques incluant une séquence codant pour une protéine d'enveloppe comprend en outre en amont de ladite séquence une séquence homologue du rétrovirus recombinant défectif utilisé, dans le système hôte-vecteur choisie notamment parmi tout ou partie du gène gag ou tout ou partie du gène pol.

6. Système selon l'une des revendications 1 à 5, caractérisé en ce que le transgène à exprimer est un gène suicide, notamment la thymidine kinase du virus Herpès Simplex (HSV1-TK).

7. Système selon l'une des revendications 1 à 5, caractérisé en ce que la séquence pseudovirale recombinante est dérivée du génome de Moloney MuLV, les séquences LTR en 5' ou en 3' étant d'origine sauvage, mutante ou combinée.

8. Système selon l'une des revendications 1 à 5, caractérisé en ce que la séquence d'acides nucléiques codant pour le gène env est d'origine virale et choisie notamment parmi les séquences codant pour le gène env du MuLV, du VSV, de HIV, de la rage.

9. Système selon l'une des revendications 1 à 5, caractérisé en ce que la séquence d'acides nucléiques codant pour le gène env est d'origine cellulaire et est constituée d'une séquence codant pour une protéine membranaire, permettant le ciblage de la particule virale sur un ligand spécifique, notamment une protéine codant pour un récepteur de type CD4.

10. Système selon l'une des revendications 1 à 5, caractérisé en ce que le gène env est une protéine chimérique dans laquelle l'extrémité carboxyterminale est dérivée des séquences intramembranaires de la proteine de l'enveloppe du Moloney.

11. Système selon l'une des revendications 1 à 10, caractérisé en ce que la séquence contenant le gène env peut être introduite ou ré-introduite par transfection par un vecteur viral, notamment l'adénovirus.

12. Système selon l'une des revendications 1 à 10 caractérisé en ce que la séquence pseudo-virale recombinante et la séquence portant le gène env. sont enveloppées dans des véhicules tels des liposomes, et introduites dans les cellules par transfection.

13. Système selon la revendication 12 caractérisé en ce que les cellules transfectables sont soit des cellules hôtes, soit des cellules cibles.

14. Système selon l'une des revendications 1 à 10, caractérisé en ce que la cellule eucaryote est une cellule établie en lignée et peut être choisie notamment parmi les lignées fibroblastiques telles la lignée 3T3, ou des lignées susceptibles d'être cultivées en suspension telles des cellules de myélomes de souris, ou des cellules VERO.

15. Système selon l'une des revendications 1 à 10, caractérisé en ce que les cellules utilisées sont des cellules de lépidoptères.

16. Procédé d'expression d'un transgène pour la thérapie génique consistant en la mise en oeuvre des étapes suivantes :
a) la construction d'un système hôte-vecteur par transfection dans une cellule eucaryote hôte, d'une part, d'une séquence pseudorétrovirale dans laquelle le gène env est délété dans sa totalité et remplacé par le transgène, notamment au niveau de l'ATG dudit gène env et, d'autre part, une séquence d'acides nucléiques comportant dans sa structure une séquence codant pour une protéine d'enveloppe sous le contrôle d'un promoteur, le cas échéant transfecté simultanément au transgène et flanqué à son extrémité 3' d'une séquence de polyadénylation,
b) la mise en contact dudit système avec les cellules dans lesquelles le transgène doit être exprimé,
c) le cas échéant, le transfert à nouveau dans les cellules cibles de la susdite séquence contenant le gène env.

17. Procédé selon la revendication 16 caractérisé en ce que les séquences pseudo-rétrovirales et la séquence portant un gène env. sont enveloppées dans des véhicules.

18. Procédé selon la revendication 17 caractérisé en ce que le véhicule de transfection est choisi parmi les liposomes, les lipides cationiques, les dérivés de polylysine, les adénovirus défectifs ou un moyen balistique.

19. Procédé selon la revendication 16 ou 17 caractérisé en ce que la séquence pseudo-rétrovirale et la séquence portant le gène env. sont portées par un même système de transfection.

20. Procédé selon la revendication 19 caractérisé en ce que le système de transfection contient en outre une ou deux séquences ITR de AAV situées en amont, ou en amont et en aval des LTR.

21. Procédé selon l'une des revendications 16 à 19, caractérisé en ce que le transgène est un gène suicide, notamment le gène de la thymidine kinase de HSV1, et en ce que les cellules dans lesquelles ledit transgène est intégré sont détruites par addition d'un analogue de nucléoside, notamment le gancyclovir ou l'acyclovir.

22. Procédé selon la revendication 16, caractérisé en ce que la séquence pseudorétrovirale est directement dérivée du virus de Moloney MuLV, les séquences LTR en 5' ou en 3', étant d'origine sauvage, mutante ou combinée.

23. Procédé selon l'une des revendications 16 à 19, caractérisé en ce que la séquence codant pour le gène env est choisie notamment parmi les séquences de virus codant pour ledit gène, notamment le MuLV, ou le VSV, le HIV, ou le virus de la rage ou des séquences codant pour des protéines de membranes cellulaires, notamment le CD4.

24. Procédé selon l'une des revendications 16 à 19, caractérisé en ce que la séquence contenant le gène env est introduite ou ré-introduite dans la cellule cible par un vecteur viral, notamment issu de l'adénovirus.

25. Procédé selon l'une des revendications 16 à 19, caractérisé en ce que la séquence contenant le gène env est introduite ou ré-introduite dans les cellules cibles par des méthodes physiques, notamment par bombardement, fusion de liposomes, micro-injection.

26. Procédé selon l'une des revendications 16 à 19, caractérisé en ce que la cellule eucaryote utilisée est choisie parmi des lignées établies n'exprimant pas le transgène d'intérêt considéré, notamment des lignées fibroblastiques 3T3 de souris, des myélomes de souris ou des cellules d'insectes, ou des cellules VERO.

27. Procédé permettant l'expression d'un transgène pour la thérapie génique qui consiste en une double transfection des cellules cibles, d'une part, par une séquence pseudo-rétrovirale recombinante dans laquelle le gène env a été délété dans sa totalité ou partiellement et substitué par ledit transgène au niveau du gène env tel que représenté dans la figure la et, d'autre part, par une séquence d'acides nucléiques contenant dans sa structure un gène enveloppe de rétrovirus, la co-expression in vivo en trans des deux séquences permettant l'expression de particules virales recombinantes infectieuses mais dépourvues dans leur génome dudit gène env.

28. Utilisation dans la fabrication d'un médicament destiné au transfert de gènes dans les cellules, notamment pour la thérapie génique, d'un système hôte-vecteur obtenu par transfection simultanée dans une cellule eucaryote, d'une part, d'une séquence pseudorétrovirale dans laquelle le gène env est délété dans sa totalité et remplacé par le transgène et, d'autre part, une séquence d'acides nucléiques comportant dans sa structure une séquence codant pour une protéine d'enveloppe sous le contrôle d'un promoteur, le cas échéant associé au transgène et flanquée à son extrémité 3' d'une séquence de polyadénylation, la cellule ainsi transfectée étant alors capable de produire des virus infectieux défectifs dépourvus du gène env.

29. Utilisation selon la revendication 28 caractérisée en ce que la séquence pseudo-rétrovirale et la séquence d'acides nucléiques portant un gène env. sont portées par un seul système de transfection.

30. Utilisation selon la revendication 29 caractérisée en ce que le système de transfection contient en outre une ou deux séquences ITR de AAV situées en amont, ou en amont et en aval des LTR.

31. Utilisation selon la revendication 28 caractérisée en ce que la séquence pseudo-rétrovirale et la séquence portant le gène env. sont portées par deux systèmes de transfection distincts.

32. Utilisation selon l'une des revendications 28 à 31, caractérisée en ce que le gène à exprimer est un gène suicide.

33. Utilisation selon la revendication 32, caractérisée en ce que le gène suicide est le gène de la thymidine kinase, les cellules cibles dans lesquelles le gène est exprimé étant détruites par addition d'un analogue de nucléoside, notamment l'acyclovir ou le gancyclovir.

34. Utilisation selon la revendication 28, caractérisée en ce que ledit système est en contact avec les cellules dans lesquelles le transgène est à exprimer, et caractérisé en ce que les virus infectieux produits par ledit système peuvent infecter lesdites cellules.

35. Utilisation selon la revendication 34, caractérisée en ce que la séquence codant pour la protéine d'enveloppe est transférée à nouveau dans lesdites cellules.

36. Médicament utilisable en thérapie génique caractérisé en ce qu'il contient comme principe actif un système de transfection contenant une séquence rétro-virale recombinante substituée au niveau du gène env. par un transgène d'intérêt, et, en dehors de la zone de régulation de l'expression des séquences rétrovirales, notamment en dehors des LTR, une séquence porteuse d'un gène env. sous la dépendance d'un promoteur.

37. Médicament selon la revendication 34 caractérisé en ce que le système de transfection contient en outre une ou deux séquences ITR de AAV situées en amont, ou en amont et en aval des LTR.

38. Médicament utilisable en thérapie génique caractérisé en ce qu'il contient comme principe actif des cellules eucaryotes ayant subi une double transfection et représentées par le système hôte-vecteur selon la revendication 1.

39. Système de transfection constitué :
- d'un vecteur viral recombinant et contenant un transgène d'intérêt dans lequel un gène essentiel à la constitution de particules virales infectieuses est substitué par ledit transgène d'intérêt,
- dudit gène essentiel sous le contrôle d'un promoteur propre, présent sur le même vecteur ou sur un vecteur distinct,
de telle façon que le produit dudit gène permette de reconstituer des particules virales défectives.

40. Système de transfection selon la revendication 39 caractérisé en ce que le vecteur viral est un vecteur rétroviral, et le transgène est substitué au niveau du gène env.

41. Système de transfection selon la revendication 39 caractérisé en ce que le vecteur viral est un vecteur adénoviral et le transgène est substitué au niveau du gène E1A.

42. Utilisation d'un système de transfection selon l'une des revendications 39 à 41 dans la fabrication d'un médicament utilisable en thérapie génique.

## Patentansprüche

1. Wirt-Vektor-System, das es ermöglicht, ein Transgen in einer Zielzelle oder einem Gewebe eines Menschen oder Tiers zu exprimieren, dadurch gekennzeichnet, daß es aus einer eukaryotischen Zelle gebildet ist und in einer Linie etabliert ist, in die transfektiert wurden:
a) eine rekombinante pseudoretrovirale Sequenz, in der das Gen env ganz oder teilweise entfernt und durch das Transgen auf Höhe des Gens env substituiert wurde, wie es in Figur 1a dargestellt ist;
b) eine Nukleinsäurensequenz, die eine kodierende Sequenz für ein Hüllprotein umfaßt, wobei die Sequenz unter dem Einfluß eines Promotors ist und an seinem 3'-Ende von einer Polyadenylierungsstelle flankiert ist, wie es in Figur 1b dargestellt ist;
wobei das rekombinante Virusgenom und die Sequenz zur trans-Komplementierung fähig sind und der Wirtszelle ermöglichen, infektiöse Viren mit Fehlstellen zu produzieren, denen das Gen env fehlt.

2. System nach Anspruch 1, dadurch gekennzeichnet, daß die Sequenz in a) und die Sequenz in b) von zwei unterschiedlichen Transfektionssystemen getragen sind.

3. System nach Anspruch 1, dadurch gekennzeichnet, daß die Sequenzen in a) und b) vom selben Transfektionssystem getragen sind, vorausgesetzt, daß die Sequenz 1b) außerhalb der Regulationszone der Expression der rekombinanten pseudoretroviralen Sequenz in a) liegt, insbesondere außerhalb der LTR.

4. System nach Anspruch 3, dadurch gekennzeichnet, daß das Transfektionssystem außerdem eine oder zwei ITR-Sequenzen von AAV umfaßt, die oberhalb oder oberhalb und unterhalb der LTR angeordnet sind.

5. System nach einem der Ansprüche 1 bis 4, dadurch gekennzeichnet, daß die Nukleinsäurensequenz, die eine für ein Hüllprotein kodierende Sequenz umfaßt, außerdem oberhalb der genannten Sequenz eine homologe Sequenz des verwendeten rekombinanten Retrovirus mit Fehlstellen aufweist, im Wirt-Vektor-System insbesondere ausgewählt aus dem ganzen oder einem Teil des Gens gag oder dem ganzen oder einem Teil des Gens pol.

6. System nach einem der Ansprüche 1 bis 5, dadurch gekennzeichnet, daß das zu exprimierende Transgen ein Suizidgen ist, insbesondere die Thymidinkinase des Virus Herpes Simplex (HSV1-TK).

7. System nach einem der Ansprüche 1 bis 5, dadurch gekennzeichnet, daß die rekombinante pseudovirale Sequenz vom Genom von Moloney MuLV abgeleitet ist, wobei die LTR-Sequenzen in 5' oder in 3' von einer Wildform, Mutante oder Kombination stammen.

8. System nach einem der Ansprüche 1 bis 5, dadurch gekennzeichnet, daß die Nukleinsäurensequenz, die für das Gen env kodiert, viralen Ursprungs ist, und insbesondere aus den Sequenzen ausgewählt ist, die für das Gen env von MuLV, VSV, HIV, Tollwut kodieren.

9. System nach einem der Ansprüche 1 bis 5, dadurch gekennzeichnet, daß die Nukleinsäurensequenz, die für das Gen env kodiert, zellulären Ursprungs ist und aus einer Sequenz besteht, die für ein Membranprotein kodiert, was es ermöglicht, den Viruspartikel auf einen spezifischen Liganden zu richten, insbesondere auf ein Protein, das für einen Rezeptor vom Typ CD4 kodiert.

10. System nach einem der Ansprüche 1 bis 5, dadurch gekennzeichnet, daß das Gen env ein Chimärenprotein ist, in dem das carboxyterminale Ende von Intramembransequenzen des Hüllproteins von Moloney abgeleitet ist.

11. System nach einem der Ansprüche 1 bis 10, dadurch gekennzeichnet, daß die das Gen env enthaltende Sequenz durch Transfektion durch einen viralen Vektor, insbesondere den Adenovirus, eingeführt oder erneut eingeführt werden kann.

12. System nach einem der Ansprüche 1 bis 10, dadurch gekennzeichnet, daß die rekombinante pseudovirale Sequenz und die das Gen env tragende Sequenz in Vehikel wie Liposome eingehüllt sind und durch Transfektion in die Zellen eingeführt werden.

13. System nach Anspruch 12, dadurch gekennzeichnet, daß die transfektierbaren Zellen entweder Wirtszellen oder Zielzellen sind.

14. System nach einem der Ansprüche 1 bis 10, dadurch gekennzeichnet, daß die eukaryotische Zelle eine in einer Linie etablierte Zelle ist und ausgewählt sein kann insbesondere aus Fibroblastenlinien wie der Linie 3T3 oder Linien, die in Suspension kultiviert werden können, wie Myelomzellen von Mäusen oder VERO-Zellen.

15. System nach einem der Ansprüche 1 bis 10, dadurch gekennzeichnet, daß die verwendeten Zellen Lepidopterenzellen sind.

16. Verfahren zur Expression eines Transgens zur Gentherapie umfassend die Ausführung der folgenden Schritte:
a) Konstruktion eines Wirt-Vektor-Systems durch Transfektion in eine eukaryotische Wirtszelle einerseits einer pseudoviralen Sequenz, in der das Gen env ganz entfernt ist und durch das Transgen ersetzt ist, insbesondere auf Höhe des ATG des Gens env, und andererseits einer Nukleinsäurensequenz, die in ihrer Struktur eine Sequenz umfaßt, die für ein Hüllprotein unter der Kontrolle eines Promotors kodiert, gegebenenfalls gleichzeitig mit dem Transgen transfektiert und an seinem 3'-Ende flankiert von einer Polyadenylierungssequenz,
b) in Kontakt bringen des Systems mit Zellen, in denen das Transgen exprimiert werden soll,
c) gegebenenfalls erneutes Übertragen der genannten Sequenz, die das Gen env enthält, in die Zielzellen.

17. Verfahren nach Anspruch 16, dadurch gekennzeichnet, daß die pseudoretroviralen Sequenzen und die Sequenz, die ein Gen env trägt, in Vehikel eingehüllt sind.

18. Verfahren nach Anspruch 17, dadurch gekennzeichnet, daß das Transfektionsvehikel ausgewählt ist aus Liposomen, kationischen Lipiden, Polylysinderivaten, Adenoviren mit Fehlstellen oder einem ballistischen Mittel.

19. Verfahren nach Anspruch 16 oder 17, dadurch gekennzeichnet, daß die pseudoretrovirale Sequenz und die Sequenz, die das Gen env trägt, durch das selbe Transfektionssystem getragen sind.

20. Verfahren nach Anspruch 19, dadurch gekennzeichnet, daß das Transfektionssystem außerdem eine oder zwei ITR-Sequenzen von AAV enthält, die oberhalb oder oberhalb und unterhalb der LTR angeordnet sind.

21. Verfahren nach einem der Ansprüche 16 bis 19, dadurch gekennzeichnet, daß das Transgen ein Suizidgen ist, insbesondere das Gen der Thymidinkinase von HSV1, und dadurch, daß die Zellen, in denen das Transgen integriert ist, durch Zugabe eines Nukleosidanalogen, insbesondere Gancyclovir oder Acyclovir, zerstört werden.

22. Verfahren nach Anspruch 16, dadurch gekennzeichnet, daß die pseudoretrovirale Sequenz direkt vom Moloney-Virus MuLV abgeleitet ist, wobei die LTR-Sequenzen in 5' und 3' von einer Wildform, Mutante oder Kombination stammen.

23. Verfahren nach einem der Ansprüche 16 bis 19, dadurch gekennzeichnet, daß die für das Gen env kodierende Sequenz insbesondere aus den Virussequenzen ausgewählt ist, die für das Gen kodieren, insbesondere MuLV oder VSV, HIV oder der Tollwutvirus oder Sequenzen, die für Zellmembranproteine, insbesondere CD4 kodieren.

24. Verfahren nach einem der Ansprüche 16 bis 19, dadurch gekennzeichnet, daß die das Gen env enthaltende Sequenz in die Zielzelle durch einen viralen Vektor, insbesondere vom Adenovirus, eingeführt oder erneut eingeführt wird.

25. Verfahren nach einem der Ansprüche 16 bis 19, dadurch gekennzeichnet, daß die das Gen env enthaltende Sequenz in die Zielzellen durch physikalische Methoden, insbesondere durch Beschießen, Liposomenfusion, Mikroinjektion eingeführt oder erneut eingeführt wird.

26. Verfahren nach einem der Ansprüche 16 bis 19, dadurch gekennzeichnet, daß die verwendete eukaryotische Zelle aus etablierten Linien ausgewählt ist, die das interessierende Transgen nicht exprimieren, insbesondere Fibroblastenlinien 3T3 von Mäusen, Myelome von Mäusen oder Insektenzellen oder VERO-Zellen.

27. Verfahren, das die Expression eines Transgens für die Gentherapie ermöglicht, das aus einer doppelten Transfektion von Zielzellen besteht, einerseits, durch eine rekombinante pseudoretrovirale Sequenz, in der das Gen env ganz oder teilweise entfernt und auf Höhe des Gens env durch das Transgen substituiert wurde, wie es in der Figur la dargestellt ist, und andererseits, durch eine Nukleinsäurensequenz, die in ihrer Struktur ein Retrovirushüllgen enthält, wobei die Coexpression in vivo der beiden Sequenzen in trans die Expression von infektiösen rekombinanten Viruspartikeln ermöglicht, die aber in ihrem Genom das Gen env nicht enthalten.

28. Verwendung eines Wirt-Vektor-Systems bei der Herstellung eines Medikaments zum Transfer von Genen in Zellen, insbesondere zur Gentherapie, das durch gleichzeitige Transfektion in eine eukaryotische Zelle erhalten wurde, einerseits, einer pseudoretroviralen Sequenz, in der das Gen env ganz entfernt und durch das Transgen ersetzt wurde und, andererseits, einer Nukleinsäurensequenz, die in ihrer Struktur eine Sequenz enthält, die für ein Hüllprotein unter der Kontrolle eines Promotors kodiert, gegebenenfalls mit dem Transgen assoziiert und an seinem 3'-Ende von einer Polyadenylierungssequenz flankiert, wobei die so transfektierte Zelle fähig ist, infektiöse Viren mit Fehlstellen ohne das Gen env zu produzieren.

29. Verwendung nach Anspruch 28, dadurch gekennzeichnet, daß die pseudoretrovirale Sequenz und die Nukleinsäurensequenz mit einem Gen env von einem einzigen Transfektionssystem getragen sind.

30. Verwendung nach Anspruch 29, dadurch gekennzeichnet, daß das Transfektionssystem außerdem eine oder zwei ITR-Sequenzen von AAV enthält, die oberhalb oder oberhalb und unterhalb der LTR angeordnet sind.

31. Verwendung nach Anspruch 28, dadurch gekennzeichnet, daß die pseudoretrovirale Sequenz und die Nukleinsäurensequenz mit dem Gen env von zwei unterschiedlichen Transfektionssystemen getragen sind.

32. Verwendung nach einem der Ansprüche 28 bis 31, dadurch gekennzeichnet, daß das zu exprimierende Gen ein Suizidgen ist.

33. Verwendung nach Anspruch 32, dadurch gekennzeichnet, daß das Suizidgen das Gen der Thymidinkinase ist, wobei die Zielzellen, in denen das Gen exprimiert wird, durch Zugabe eines Nukleosidanalogen, insbesondere von Acyclovir oder Gancyclovir zerstört werden.

34. Verwendung nach Anspruch 28, dadurch gekennzeichnet, daß das System mit den Zellen in Kontakt ist, in denen das Transgen zu exprimieren ist, und dadurch gekennzeichnet, daß die durch das System produzierten infektiösen Viren die genannten Zellen infizieren können.

35. Verwendung nach Anspruch 34, dadurch gekennzeichnet, daß die für das Hüllprotein kodierende Sequenz erneut in die Zellen transferiert wird.

36. Medikament verwendbar in der Gentherapie, dadurch gekennzeichnet, daß es als aktiven Wirkstoff ein Transfektionssystem enthält, das eine rekombinante Retrovirussequenz enthält, die auf Höhe des Gens env durch ein interessierendes Transgen substituiert ist, und außerhalb der Zone zur Regulation der Expression der retroviralen Sequenzen, insbesondere außerhalb der LTR, eine Trägersequenz eines Gens env unter der Abhängigkeit eines Promotors.

37. Medikament nach Anspruch 36, dadurch gekennzeichnet, daß das Transfektionssystem außerdem eine oder zwei ITR-Sequenzen von AAV enthält, die oberhalb oder oberhalb und unterhalb der LTR angeordnet sind.

38. Medikament verwendbar in der Gentherapie, dadurch gekennzeichnet, daß es als aktiven Wirkstoff eukaryotische Zellen enthält, die einer doppelten Transfektion unterzogen wurden und durch das Wirt-Vektor-System nach Anspruch 1 dargestellt sind.

39. Transfektionssystem gebildet aus:
- einem rekombinanten viralen Vektor und das ein interessierendes Transgen enthält, in dem ein für die Bildung von infektiösen Viruspartikeln essentielles Gen durch das interessierende Transgen substituiert ist,
- dem essentiellen Gen unter der Kontrolle eines geeigneten Promotors, der auf demselben Vektor oder auf einem anderen Vektor vorhanden ist,
in der Weise, daß das Produkt des Gens es ermöglicht, Viruspartikel mit Fehlstellen zu rekonstituieren.

40. Transfektionssystem nach Anspruch 39, dadurch gekennzeichnet, daß der virale Vektor ein retroviraler Vektor ist und das Transgen auf Höhe des Gens env substituiert ist.

41. Transfektionssystem nach Anspruch 39, dadurch gekennzeichnet, daß der virale Vektor ein Adenovirusvektor ist und das Transgen auf Höhe des Gens E1A substituiert ist.

42. Verwendung eines Transfektionssystems nach einem der Ansprüche 39 bis 41 bei der Herstellung eines Medikaments, das in der Gentherapie verwendbar ist.

## Claims

1. Host-vector system which makes it possible to express a transgene in a target cell or a human or animal tissue, characterized in that it consists of a eukaryotic cell established as a line, into which there have been transfected:
a) a recombinant pseudo-retroviral sequence in which the env. gene has been deleted totally or partially and substituted by the said transgene at the level of the env. gene as represented in Figure 1a;
b) a nucleic acid sequence including a sequence encoding an envelope protein which sequence is in dependence on a promoter and is flanked at its 3' end with a polyadenylation site, as represented in Figure 1b;
the said recombinant viral genome and the said sequence being capable of trans-complementing each other and allowing the said host cell to produce defective infectious viruses lacking the env. gene.

2. System according to Claim 1, characterized in that the sequence in a) and the sequence in b) are carried by two separate transfection systems.

3. System according to Claim 1, characterized in that the sequences in a) and in b) are carried by the same transfection system provided that the sequence 1b) is outside the region for regulating the expression of the recombinant pseudo-retroviral sequence in a), in particular outside the LTRs.

4. System according to Claim 3, characterized in that the transfection system contains, in addition, one or two AAV ITR sequences situated upstream, or upstream and downstream of the LTRs.

5. System according to one of claims 1 to 4, characterized in that the nucleic acid sequence including a sequence encoding an envelope protein comprises, in addition, upstream of the said sequence, a homologous sequence of the defective recombinant retrovirus used in the host-vector system, chosen especially from all or part of the gag gene or all or part of the pol gene.

6. System according to one of claims 1 to 5, characterized in that the transgene to be expressed is a suicide gene, especially the Herpes Simplex virus thymidine kinase (HSV1-TK).

7. System according to one of claims 1 to 5, characterized in that the recombinant pseudoviral sequence is derived from the moloney genome MuLV, the LTR sequences in 5' or in 3' being of wild-type, mutant or combined origin.

8. System according to one of Claims 1 to 5, characterized in that the nucleic acid sequence encoding the env. gene is of viral origin and is chosen especially from the sequences encoding the MuLV, VSV, HIV and rabies env. gene.

9. System according to one of claims 1 to 5, characterized in that the nucleic acid sequence encoding the env. gene is of cellular origin and consists of a sequence encoding a membrane protein, allowing the targeting of the viral particle on a specific ligand, especially a protein encoding a CD4-type receptor.

10. System according to one of claims 1 to 5, characterized in that the env. gene is a chimeric protein in which the carboxy-terminal end is derived from intra-membrane sequences of the Moloney envelope protein.

11. System according to one of claims 1 to 10, characterized in that the sequence containing the env. gene may be introduced or reintroduced by transfection with a viral vector, especially an adenovirus.

12. System according to one of claims 1 to 10, characterized in that the recombinant pseudo-viral sequence and the sequence carrying the env. gene are enveloped in vehicles such as liposomes, and introduced into the cells by transfection.

13. System according to claim 12, characterized in that the transfectable cells are either host cells, or target cells.

14. System according to one of claims 1 to 10, characterized in that the eukaryotic cell is a cell established as a line and may be chosen especially from the fibroblast lines such as the 3T3 line, or lines capable of being cultured in suspension, such as mouse myeloma cells, or VERO cells.

15. System according to one of Claims 1 to 10, characterized in that the cells used are Lepidoptera cells.

16. Process for expressing a transgene for gene therapy which consists in using the following steps:
a) construction of a host-vector system by transfection, into a eukaryotic host cell, on the one hand, of a pseudo-retroviral sequence in which the env. gene is deleted totally and replaced with the transgene, especially at the level of the ATG of the said env. gene and, on the other hand, a nucleic acid sequence containing, in its structure, a sequence encoding en envelope protein under the control of a promoter, where appropriate transfected simultaneously with the transgene and flanked at its 3' end by a polyadenylation sequence,
b) bringing the said system into contact with the cells in which the transgene has to be expressed,
c) where appropriate, again transferring, into the target cells, the abovementioned sequence containing the env. gene.

17. Process according to claim 16, characterized in that the pseudo-retroviral sequences and the sequence carrying an env. gene are enveloped in vehicles.

18. Process according to claim 17, characterized in that the transfection vehicle is chosen amongst liposomes, cationic lipids, polylysine derivatives, defective adenoviruses or a ballistic means.

19. Process according to claim 16 or 17, characterized in that the pseudo-retroviral sequence and the sequence carrying the env. gene are carried by the same transfection system.

20. Process according to claim 19, characterized in that the transfection system contains in addition one or two AAV ITR sequences situated upstream, or upstream and downstream of the LTRs.

21. Process according to one of Claims 16 to 19, characterized in that the transgene is a suicide gene, especially the HSV1 thymidine kinase gene, and in that the cells into which the said transgene is integrated are destroyed by addition of a nucleoside analog, especially gancyclovir or acyclovir.

22. Process according to claim 16, characterized in that the pseudo-retroviral sequence is directly derived from the Moloney virus MuLV, the LTR sequences in 5' or in 3' being of wild-type, mutant or combined origin.

23. Process according to one of claims 16 to 19, characterized in that the sequence encoding the env. gene is chosen especially from the sequences of viruses encoding the said gene, especially MuLV, or VSV, HIV, or the rabies virus or sequences encoding cell membrane proteins, especially CD4.

24. Process according to one of claims 16 to 19, characterized in that the sequence containing the env. gene is introduced or reintroduced into the target cell by a viral vector, especially derived from an adenovirus.

25. Process according to one of claims 16 to 19, characterized in that the sequence containing the env. gene is introduced or reintroduced into the target cells by physical methods, especially by bombardment, fusion of liposomes, microinjection.

26. Process according to one of claims 16 to 19, characterized in that the eukaryotic cell used is chosen from established lines not expressing the transgene of interest considered, especially mouse 3T3 fibroblast lines, mouse myelomas or insect cells, or VERO cells.

27. Process allowing the expression of a transgene for gene therapy which consists in a double transfection of the target cells, on the one hand, with a recombinant pseudo-retroviral sequence in which the env. gene has been deleted totally or partially and substituted by the said transgene at the level of the env. gene as represented in Figure la and, on the other hand, with a nucleic acid sequence containing in its structure a retrovirus envelope gene, the in vivo co-expression in trans of the two sequences allowing the expression of infectious recombinant viral particles, but lacking the said env. gene in their genome.

28. Use, in the manufacture of a medicinal product for gene transfer to cells, in particular for gene therapy, of a host-vector system obtained by simultaneous transfection, into a eukaryotic host cell, on the one hand, of a pseudo-retroviral sequence in which the env. gene is deleted totally and replaced with the transgene and, on the other hand, a nucleic acid sequence containing, in its structure, a sequence encoding an envelope protein under the control of a promoter, where appropriate, combined with the transgene and flanked at its 3' end by a polyadenylation sequence, the cell thus transfected being then capable of producing defective infectious viruses lacking the env. gene.

29. Use according to claim 28, characterized in that the pseudo-retroviral sequence and the nucleic acid sequence carrying an env. gene are carried by a single transfection system.

30. Use according to claim 29, characterized in that the transfection system contains, in addition, one or two AAV ITR sequences situated upstream, or upstream and downstream of the LTRs.

31. Use according to claim 28, characterized in that the pseudo-retroviral sequence and the sequence carrying the env. gene are carried by two separate transfection systems.

32. Use according to one of claims 28 to 31, characterized in that the gene to be expressed is a suicide gene.

33. Use according to claim 32, characterized in that the suicide gene is the thymidine kinase gene, the target cells in which the gene is expressed being destroyed by addition of nucleoside analog, especially acyclovir or gancyclovir.

34. Use according to claim 28, characterized in that said system is in contact with the cells in which the transgene has to be expressed, and characterized in that the infectious viruses produced by said system are able to infect said cells.

35. Use according to claim 34, characterized in that the sequence coding for the envelope protein is again transferred into said cells.

36. Medicinal product which can be used in gene therapy, characterized in that it contains, as active ingredient, a transfection system containing a recombinant retroviral sequence substituted, at the level of the env. gene, by a transgene of interest, and outside the region for regulating the expression of the retroviral sequences, especially outside the LTRs, a sequence carrying an env. gene in dependence on a promoter.

37. Medicinal product according to claim 34, characterized in that the transfection system contains, in addition, one or two AAV ITR sequences situated upstream, or upstream and downstream of the LTRs.

38. Medicinal product which can be used in gene therapy, characterized in that it contains, as active ingredient, eukaryotic cells which have been subjected to a double-transfection and represented by the host-vector system according to claim 1.

39. Transfection system consisting of:
- a recombinant viral vector containing a transgene of interest in which a gene essential for the constitution of infectious viral particles is substituted by said transgene of interest,
- said essential gene under the control of a particular promoter, present on the same vector, or a different vector,
- such that the product of this gene makes it possible to reconstitute defective viral particles.

40. Transfection system according to claim 39, characterized in that the viral vector is a retroviral vector and the transgene is substituted at the level of the env. gene.

41. Transfection system according to claim 39, characterized in that the viral vector is an adenoviral vector and the transgene is substituted at the level of the E1A gene.

42. Use of a transfection system according to one of Claims 39 to 41, in the manufacture of a medicinal product which can be used in gene therapy.
